# EUROPEAN PATENT APPLICATION

(11) **EP 4 483 864 A1**
(43) Date of publication of application: **01.01.2025**
(21) Application number: 23182485.5
(22) Date of filing: 29.06.2023
(51) Int. Cl.: A61K 9/16, A61K 9/50, A61K 45/06

(54) **COMPOSITION AND METHOD FOR PRETREATING CANCER**

(71) Applicant: Valcuria AB, 223 81 Lund (SE)
(72) Inventor: DROTT, Johan, 223 50 LUND (SE); NICKLASSON, Fredrik, 237 33 BJÄRRED (SE); HORLER, Andrew, RUDDINGTON, NG11 6NY (GB); PANCHOLI, Mehul, DIDCOT, OX11 7XB (GB)
(74) Representative: Brann AB

(57) **Abstract**

The present invention relates to a pharmaceutical composition for oral administration, the pharmaceutical composition comprising a) immediate release granules comprising i. an active ingredient selected from the group consisting of valproic acid, valproate semisodium, sodium valproate and magnesium valproate, and ii. a filler, and b) sustained release pellets comprising: i. pellet cores comprising: (1) an active ingredient selected from the group consisting of valproic acid, valproate semisodium, sodium valproate, and magnesium valproate, and (2) a filler, ii. a subcoat provided on the pellet cores, the content of the subcoat being 10-20 wt% based on the weight of the pellet cores, and the subcoat comprising a film forming agent, and iii. a sustained release coating provided on the subcoat, the content of the sustained release coating being 25-100 wt% based on the weight of the pellet cores coated with the subcoat, and the sustained release coating comprising a film forming agent, wherein the amount of active ingredient in the immediate release granules makes up 70-80 wt% of the total weight of active ingredient in the pharmaceutical composition, and the amount of active ingredient in the sustained release pellets makes up 20-30 wt% of the total weight of active ingredient in the pharmaceutical composition. In further aspects the present invention relates to a method of producing the pharmaceutical composition and the pharmaceutical composition for use in a method of pretreating cancer.

## Description

### FIELD OF INVENTION

The invention relates to the field of compositions and methods of pretreating cancer, i.e., where one or more compositions are administered to an individual in need of cancer treatment as a pretreatment prior to other treatments to enhance the effect, or mitigate the side-effects, of the treatments.

### BACKGROUND OF INVENTION

Cancer can be defined as an abnormal growth of cells which exhibits signs of uncontrolled proliferation and disturbed programmed cell death. From a classical view, sequential genetic events lead to malignant transformation, resulting in a cell clone that does not respect the integrity of other cells and tissues, and may eventually metastasize. Cancer can involve any tissue of the body and have many different forms in each body area.

Malignant lymphoma can be defined as a malignant transformation of the lymphatic cells of the hematopoietic system. Lymphomas can be divided into aggressive lymphomas and indolent lymphomas. Aggressive lymphomas are characterized by a rapid growth pattern and can have dramatic clinical features. However, aggressive lymphomas can reach a complete cure by treatment with chemotherapy, radiotherapy, and monoclonal antibodies. In contrast, indolent lymphomas (e.g., follicular lymphomas) have a slow growth pattern, and usually a more modest clinical presentation. However, although indolent lymphomas cannot reach a complete cure by standard lymphoma treatment, they can sometimes be cured by allogeneic stem cell transplantation. The median survival time for humans afflicted by follicular lymphomas is 8-10 years. Diffuse Large B Cell Lymphoma and Hodgkin lymphoma belong to the group of aggressive lymphomas, while follicular lymphoma and chronic lymphocytic leukaemia are indolent lymphomas. Myelomas consist of malignantly transformed plasmacells. They are related to indolent lymphomas but are usually considered an entity of their own. The prognosis is pessimistic, with a median survival time of 5-7 years.

One of the most frequent subtypes of malignant lymphoma is Diffuse Large B-cell Lymphoma (DLBCL) with an incidence of about 500 cases/year in Sweden. DLBCLs constitute 60-70% of the group of aggressive lymphomas. The median age at diagnosis is 70 years, and DLBCL is slightly more common in males than in females.

Standard first line treatment of DLBCL is chemotherapy consisting of a combination of cyclophosphamide, doxorubicin, vincristine, and prednisone (CHOP). During recent years, addition of the CD20 antibody rituximab has become international clinical standard (R-CHOP), leading to improved progression-free, event-free, disease-free and overall survival (Morrison, Expert Rev Anticancer Ther, 2008; 8(10): pp. 1651-1658). Still, since as many as 45 % of patients die from their disease, there is a pronounced clinical need to increase progression-free survival in DLBCL patients.

One important field in the study of cancer diseases is the regulation of DNA transcription. This is a complex process and the mechanisms involved are only partially known. Histone Deacetylases (HDACs) can regulate expression of tumour suppressor genes and activities of transcription factors involved in both cancer initiation and progression. HDACs act through alteration of either DNA or the structural components of chromatin by histone deacetylation, thus affecting the three-dimensional conformation of DNA without changing or interrupting its sequence (epigenetic modifications). It has also been suggested that they may alter the sensitivity to DNA damaging chemotherapy through modulation of chromatin structure. Along these lines, several in vitro studies have suggested that inhibition of HDACs can synergize with chemotherapy.

Therefore, numerous HDAC inhibitors have been developed during the recent years. They can be divided into four classes: hydroxamic acids/carbamic acids, cyclic peptides, aliphatic acids and benzamides. Examples of HDAC inhibitors which have been approved for treatment of cancer include vorinostat and romidepsin, which are approved for the treatment of cutaneous T-cell lymphoma by the FDA (Food and drug administration), and which are currently evaluated in the treatment of other malignancies.

The clinically most well-known HDAC inhibitor is the anticonvulsant valproic acid, which has been utilized in the treatment of epilepsy since the 1970s. Valproic acid belongs to the aliphatic acid class of inhibitors.

The present inventors have previously shown that HDAC inhibitors in combination with steroids are useful when being administered to a human in need of cancer treatment as a pretreatment prior to other treatments, the result being that the effect of the treatment is enhanced, see WO2012/128709.

Despite the promise of more effective treatment of cancer made by the advent of pretreatment using a HDAC inhibitor as taught in WO2012/128709, there is still a need for further development.

One difficulty that arises is that of how to effectively administer the HDAC inhibitor to meet the requirements of a pretreatment use of the HDAC inhibitor. One HDAC inhibitor, valproic acid, has been in clinical use since the 1970s, but this clinical use has been related to a different medical field, i.e., that of treating of epilepsy, and not to the new field of pretreatment for cancer. Consequently, earlier obtained methods of how to administer an HDAC inhibitor, such as valproic acid or its derivatives, and the compositions then used, need not be the most effective when administering the HDAC inhibitor, such as valproic acid or its derivatives, as a pretreatment for cancer.

Accordingly, it is an object of the present invention to provide a pharmaceutical composition that provides an efficient pretreatment of cancer.

It is a further object of the present invention to provide a pharmaceutical composition that may be produced in a simple and efficient way.

It is a further object of the present invention to provide a method of producing the pharmaceutical composition.

It is yet a further object of the present invention to provide a pharmaceutical composition for use in a method of pretreating cancer.

It is still a further object of the present invention to provide a method of pretreating cancer by administering the pharmaceutical composition.

### SUMMARY OF THE INVENTION

At least one of the above-mentioned objects are, according to the corresponding first and second aspects of the present invention, achieved by a pharmaceutical composition for oral administration, the pharmaceutical composition comprising:
a) immediate release granules comprising:
   i. an active ingredient selected from the group consisting of valproic acid, valproate semisodium, sodium valproate, and magnesium valproate, and
   ii. a filler,
b) sustained release pellets, comprising:
   i. pellet cores comprising:
      (1) an active ingredient selected from the group consisting of valproic acid, valproate semisodium, sodium valproate, and magnesium valproate, and
      (2) a filler,
   ii. a subcoat provided on the pellet cores, the content of the subcoat being 10-20 wt% based on the weight of the pellet cores, and the subcoat comprising a film forming agent, and
   iii. a sustained release coating provided on the subcoat, the content of the sustained release coating being 25-100 wt% based on the weight of the pellet cores coated with the subcoat, and the sustained release coating comprising a film forming agent,

wherein the amount of active ingredient in the immediate release granules makes up 70-80 wt% of the total weight of active ingredient in the pharmaceutical composition, and the amount of active ingredient in the sustained release pellets makes up 20-30 wt% of the total weight of active ingredient in the pharmaceutical composition, and,
a method of producing the pharmaceutical composition according to any preceding claim, comprising the steps of:
   i. producing the immediate release granules,
   ii. producing the pellet cores,
   iii. coating the pellet cores with the subcoat by performing the substeps of:
      a. suspending or dissolving the subcoat in aqueous media to form an aqueous solution or dispersion of the subcoat,
      b. contacting the pellet cores with the aqueous solution or dispersion of the subcoat to produce pellet cores provided with the subcoat,
   iv. further coating the pellet cores provided with the subcoat with a sustained release coating by performing the substeps of
      a. suspending or dissolving the sustained release coating in aqueous media to form an aqueous solution or dispersion of the sustained release coating, and
      b. contacting the pellet cores, coated with the subcoat, with the aqueous solution or dispersion of the sustained release coating to produce sustained release pellets.

The present invention is thus based on the discovery that a subcoat, when provided on the pellet cores, ensures that the later application of the sustained release coating, which sustained release coating is advantageously applied using an aqueous solution or dispersion, does not interfere with the structure of the pellet cores and therefore does not lead to unexpected release rates of active ingredient from the sustained release pellets.

In other words, as was found in Example 1, the direct application of a sustained release coating to pellet cores could lead to unexpected release rates of the active ingredient. As found, this unexpected release rate could be attributed to the aqueous solution or dispersion used to apply the sustained release coating. Specifically, it was found that the high solubility of the API and the swelling nature of MCC, combined with the porosity of the sustained release coating, allowed water to penetrate the coating into the pellet core, which caused the core to swell and rupture and thus provide a rapid release of all active ingredient.

The problem of the unexpected release rate was solved by the present inventors when a subcoat was applied to the pellet cores prior to the application of the sustained release coating. The subcoat thus appears to protect the pellet cores from the aqueous solution or dispersion, so that the sustained release coating can be applied using an aqueous solution or dispersion.

Further, as shown in example 2, the pharmaceutical composition according to the first aspect of the present invention has several advantages of known compositions. Among these advantages are a lower maximum plasma concentration (Cₘₐₓ) of the active ingredient, less difference between plasma concentration profiles for fed and fasted state administration, respectively, a lower risk of accumulation of the active ingredient, and a more stable exposure with maintained reduced maximum concentrations of the active ingredient even when the pharmaceutical composition is administered less frequently, such as twice daily. This differed from the reference composition which, if taken twice daily with the corresponding dose, would appear to give a higher Cₘₐₓ.

Finally, as shown in example 3, the pharmaceutical composition according to the first aspect of the present invention provides a higher extent of histone deacetylation inhibition than the known composition.

A third aspect of the present invention relates to the pharmaceutical composition according to the first aspect of the present invention for use in a method of pretreating cancer, wherein the pharmaceutical composition is administered to a human suffering from cancer.

### BRIEF DESCRIPTION OF THE DRAWINGS

Fig 1A, 1B, and 1C show dissolution results for the sustained release pellets with varying amounts of subcoat, sustained release coating, pore former, and drying method.
Fig 1D shows dissolution profiles for the finished pharmaceutical composition.
Fig. 2A shows the mean (± standard deviation) plasma concentration of total valproate (µM) vs nominal time (h),
Fig. 2B shows the data in Fig. 2A in a semi logarithmic graph,
Fig. 3A shows the mean (± standard deviation) plasma concentration of free (unbound) valproate (µM) vs nominal time (h),
Fig. 3B shows the data in Fig. 3A in a semi logarithmic graph,
Fig. 4A shows the mean (± standard deviation) plasma concentration of total valproate (µM) vs nominal time (h) for a twice daily dosing of 30 mg valproate per kg body weight,
Fig. 4B shows the mean (± standard deviation) plasma concentration of free valproate (µM) vs nominal time (h) for a thrice daily dosing of 20 mg valproate per kg body weight, and
Fig. 5 shows a boxplot of acetylation of lysine 9 of lysine H3 (acH3K9) in peripheral blood mononuclear cells (PBMCs) compared to base line expression.

### DETAILED DESCRIPTION

The corresponding first and second aspects of the present invention relate to a pharmaceutical composition for oral administration, the pharmaceutical composition comprising:
a) immediate release granules comprising:
   i. an active ingredient selected from the group consisting of valproic acid, valproate semisodium, sodium valproate, and magnesium valproate, and
   ii. a filler,
b) sustained release pellets, comprising:
   i. pellet cores comprising:
      (1) an active ingredient selected from the group consisting of valproic acid, valproate semisodium, sodium valproate, and magnesium valproate, and
      (2) a filler,
   ii. a subcoat provided on the pellet cores, the content of the subcoat being 10-20 wt% based on the weight of the pellet cores, and the subcoat comprising a film forming agent, and
   iii. a sustained release coating provided on the subcoat, the content of the sustained release coating being 25-100 wt% based on the weight of the pellet cores coated with the subcoat, and the sustained release coating comprising a film forming agent,

wherein the amount of active ingredient in the immediate release granules makes up 70-80 wt% of the total weight of active ingredient in the pharmaceutical composition, and the amount of active ingredient in the sustained release pellets makes up 20-30 wt% of the total weight of active ingredient in the pharmaceutical composition,
   and
a method of producing the pharmaceutical composition according to any preceding claim, comprising the steps of:
   i. producing the immediate release granules,
   ii. producing the pellet cores,
   iii. coating the pellet cores with the subcoat by performing the substeps of:
      a. suspending or dissolving the subcoat in aqueous media to form an aqueous solution or dispersion of the subcoat,
      b. contacting the pellet cores with the aqueous solution or dispersion of the subcoat to produce pellet cores provided with the subcoat,
   iv. further coating the pellet cores provided with the subcoat with a sustained release coating by performing the substeps of
      a. suspending or dissolving the sustained release coating in aqueous media to form an aqueous solution or dispersion of the sustained release coating, and
      b. contacting the pellet cores, coated with the subcoat, with the aqueous solution or dispersion of the sustained release coating to produce sustained release pellets.

The pharmaceutical composition is for oral administration, i.e., it is suitable for oral administration. In other words, the pharmaceutical composition is formulated to be taken orally by a subject. The pharmaceutical composition may be suitable for oral administration by being packaged so as to allow the immediate release granules and the sustained release pellets to be swallowed or ingested by a subject. The pharmaceutical composition may comprise a capsule containing the immediate release granules and the sustained release pellets. The capsule may be sized to be swallowable and may be made of a material that erodes in the stomach or gut so as to release the immediate release granules and the sustained release pellets. As an alternative to a capsule, the pharmaceutical composition may comprise a sachet or a bag holding the immediate release granules and the sustained release pellets. The sachet or a bag may be made of a material that erodes in the stomach or gut so as to release the immediate release granules and the sustained release pellets. Alternatively, the capsule, sachet or bag may be opened to release the immediate release granules and the sustained release pellets, whereafter the immediate release granules and the sustained release pellets are ingested directly.

The immediate release granules and the sustained release pellets may alternatively be packaged in separate capsules, sachets, or bags, each of which may be swallowed or ingested, or opened to release the immediate release granules and the sustained release pellets, as described above.

The pharmaceutical composition is suitable for pretreating cancer, wherein the cancer is preferably selected from the croup consisting of diffuse large B cell lymphoma (DLBCL), follicular lymphoma, chronic lymphocytic leukaemia, T cell lymphoma, myeloma, and Hodgkin lymphoma.

The immediate release granules are granules configured to release the active ingredient immediately, or at least shortly after ingestion. This provides a fast or sudden release of the active ingredient and a correspondingly fast uptake of the active ingredient into the blood stream of the subject. This decreases the time needed for reaching an effective concentration of the active ingredient in the bloodstream of the subject following administration of the pharmaceutical composition.

Preferably, the immediate release granules have a release of at least 75 wt%, such as at least 80 wt%, preferably at least 95 wt%, such as 100 wt%, of the active ingredient contained therein within 15 min in pH 1 medium.

The release of active ingredient in pH 1 medium was tested as follows: immediate release granules were placed in a basket which was lowered into 0.1N HCl medium. Samples were taken at 5, 15, 30, 45 and 60 minutes to determine how much of the active ingredient had been released from the immediate release granules. During the test, the medium was stirred using a paddle or by rotating the basket. Stirring (paddle or basket) speed was 100 rpm.

The number of immediate release granules in the pharmaceutical composition depends on the amount of active ingredient in each granule, the total amount of active ingredient in the pharmaceutical composition, and the ratio of the amount of active ingredient in immediate release granules compared to the amount of active ingredient in the sustained release pellets. Typically, the pharmaceutical composition contains a plurality of immediate release granules. The immediate release granules are typically not spherical.

The active ingredient is selected from the group consisting of valproic acid, valproate semisodium, sodium valproate, and magnesium valproate.

Valproic acid is a histone deacetylation inhibitor, i.e., and a HDAC inhibitor. As shown in WO2012/128709, HDAC inhibitors, especially in combination with steroids, are useful when being administered to a subject in need of cancer treatment as a pretreatment prior to other treatments.

Chemically, valproic acid (VPA) is an organic weak acid. The conjugate base is valproate. The sodium salt of the acid is sodium valproate, the magnesium salt of the acid is magnesium valproate, and the coordination complex of valproic acid and sodium valproate is known as valproate semisodium. The latter is marketed under the various brand names Depakote, Depakote ER, Depakene, Depacon, Depakine, Valparin, Stavzor and Ergenyl. Sodium valproate is marketed in Sweden as Absenor, Depakine, and Orfiril. Valproic acid is marketed in Sweden as Ergenyl and Depakine.

The filler has the purpose of providing a matrix with which the active ingredient can be mixed. The filler further assists in providing a compactable mass so that the immediate release granules can be produced. The filler may for example comprise or consist of cellulose, such as preferably microcrystalline cellulose, and/or calcium phosphate.

The sustained release pellets are pellets configured to release the active ingredients over a sustained period of time.

Preferably the sustained release pellets have a release of the active ingredient contained therein of 95-100%, preferably 100%, within 8 hours at pH 6.8.

Additionally, the sustained release pellets preferably have a release of the active ingredient contained therein of 55-65%, preferably 60%, within 3 hours at pH 6.8. Further additionally, the sustained release pellets preferably have a release of the active ingredient contained therein of 25-35%, preferably 30%, within 2 hours.

The release of active ingredient in pH 6.8 medium was tested as follows: sustained release pellets (100 mg) were placed in a basket which was placed in 0.1N HCl medium for 15 min before the basket was taken out. The vessel holding the pH 1 medium was cleaned and filled with pH 6.8 phosphate buffer solution equilibrated at 37°C. Samples were taken at 30 minutes, 1, 2, 3, 5, 8 and 12 hours. During the test, the medium was stirred using a paddle or by rotating the basket. Stirring (paddle or basket) speed was 100 rpm during both stages of the test, and a final infinity spin at 250 rpm for 15 minutes ended the test.

The term sustained is to be understood as encompassing the term extended. Within the context of the present invention, the terms granule and pellet are used to differentiate the immediate release part of the pharmaceutical composition from the sustained release part of the pharmaceutical composition. Accordingly, a sustained release pellet may have the same size and shape, and may be formed by the same process, as a granule. Generally, however, the immediate release granules are smaller than the sustained release pellets. Further, the immediate release granules may be less spherical than the sustained release pellets. Yet further, the immediate release granules may be formed by other processes than the sustained release pellets.

The pellet cores are the innermost parts of the sustained release pellets. The pellets cores are formed from the active ingredient and a filler, and as such generally can release the active ingredient rapidly or immediately. It is by the application of primarily the sustained release coating that the pellet cores become the sustained release pellets because the sustained release coating slows down release of the active ingredient from the pellet cores.

As noted, the active ingredient in the pellet cores is selected among the same active ingredients as found in the immediate release granules. Preferably the active ingredient is the same in both the immediate release granules and the pellet cores of the sustained release pellets, but the active ingredients may alternatively be different. Further, the active ingredient may comprise a mixture of the listed forms of valproic acid, and the immediate release granules and the pellet cores of the sustained release pellets may comprise different active ingredients or different mixtures of active ingredients.

Preferably however, the active ingredient is sodium valproate in both the immediate release granules and the pellet cores of the sustained release pellets.

In a similar way as the filler in the immediate release granules, the filler in the pellet cores has the purpose of providing a matrix with which the active ingredient can be mixed. The filler further assists in providing a mass suitable for processing so that the pellet cores can be produced. The filler may for example comprise or consist of cellulose, such as preferably microcrystalline cellulose, and/or calcium phosphate.

The subcoat is provided on the pellet cores. In other words, the surface of the pellet cores is at least partially, preferably fully, covered by the subcoat. The subcoat is thus applied between the surface of the pellet cores and the sustained release coating. The subcoat has the purpose, as evident from the examples, of ensuring that the sustained release coating can be applied using an aqueous solution or dispersion. Without wishing to be bound by theory, it appears that the presence of the subcoat prevents excessive wetting, swelling and rupture of the pellet cores and subsequent release of the active ingredient when the sustained release coating is applied by contacting the pellet cores with the aqueous solution or dispersion containing the sustained release coating. The subcoat is thus inter alia configured to limit or decrease wetting of the pellet cores during application of the sustained release coating.

Using the subcoat thus allowed the sustained release coating to be applied with an aqueous solution or dispersion. A further effect obtained by the subcoat is to smoothen the surface of the pellet cores. This also assists in ensuring a good coating to the pellet cores with the sustained release coating because the sustained release coating then has a smoother surface to adhere to. Further, the smoother surface provided by the subcoat allows the sustained release coating to be applied in a more uniform layer thickness which further increases the uniformity of the sustained release coating and the release rate of the sustained release pellets. The subcoat is provided in a content of 10-20 wt% based on the weight of the pellet cores. This range has been found to provide the desired protection against wetting of the pellet cores while maintaining a sufficiently low total content of subcoat so as to not lower the content of active ingredient in the pellet cores unnecessarily and/or increasing the size of the sustained release pellets unnecessarily. The film forming agent may be any agent that, when mixed with an aqueous solution and contacted with the pellet core, forms a film or coating on the pellet core. The film forming agent may for example comprise cellulose such as hydroxypropyl cellulose and/or hydroxypropyl methylcellulose. Preferably the subcoat and/or the film forming agent is more or less hydrophobic. An increased hydrophobicity may be obtained by increasing the number of aliphatic groups in the film forming agent, i.e. by replacing polar hydroxyl groups of cellulose with non-polar substituents, as illustrated by the increased number of aliphatic groups in hydroxypropyl cellulose and hydroxypropyl methylcellulose compared to cellulose. Alternatively, the increased hydrophobicity may be obtained by providing hydrophobic compounds or substances such as a mineral such as talc (magnesium silicate). Preferably the subcoat comprises hydroxypropyl cellulose and/or hydroxypropyl methylcellulose as film forming agents. Preferably the subcoat consists of one or more film forming agents and one or more hydrophobic compounds or substances.

The sustained release coating is provided on the subcoat. This means that the sustained release coating at least partially, preferably fully, covers the subcoat, and thereby the pellet cores. The sustained release coating has the purpose of eroding slowly in the small intestine so as to allow the active ingredient to slowly release from the sustained release pellets. This ensures a steady and slow release of the active ingredient, and accordingly a steady and slow uptake of the active ingredient into the subject's bloodstream. This helps maintaining a concentration of the active ingredient in the bloodstream that is at or above the effective concentration, between administrations of the pharmaceutical composition.

The sustained release coating is provided in a content of 25-100 wt% based on the weight of the pellet cores coated with the subcoat. This range has been found to provide the desired release profile for the sustained release pellets. The film forming agent may be any agent that, when mixed with an aqueous solution and contacted with the pellet core coated with the subcoat, forms a film or coating on the subcoat. The film forming agent may for example comprise cellulose such as ethylcellulose. Preferably the film forming agent of the sustained release coating is different from the film forming agent of the subcoat. Preferably, the film forming agent in the sustained release coating is more hydrophobic, i.e. has a higher content of aliphatic groups, compared to the film forming agent in the subcoat. As an example, the sustained release coating may preferably comprise ethylcellulose, which has a higher content of aliphatic groups compared to hydroxypropyl cellulose and hydroxypropyl methylcellulose preferred in the subcoat.

The sustained release coating may further comprise a pore former at an amount of 0-10%, preferably 0-5%, more preferably 1-4%, such as 2-3%. By providing a pore former in the sustained release coating, the rate of release of active ingredient from the sustained release pellets may be further adjusted.

A pore former is an agent that is more hydrophilic than the film forming agent of the sustained release coating. The pore-former is dispersed in the sustained release coating and, by its higher hydrophilicity, forms pores in the sustained release coating when the sustained release coating comes in contact with aqueous media.

Preferably the sustained release coating comprises ethylcellulose as film forming agent. Preferably the sustained release coating consists of one or more film forming agents and, optionally, one or more pore formers.

The amount of active ingredient in the immediate release granules makes up 70-80 wt% of the total weight of active ingredient in the pharmaceutical composition, and the amount of active ingredient in the sustained release pellets makes up 30-20 wt% of the total weight of active ingredient in the pharmaceutical composition. The sum of the percentages is 100 wt%. In other words, 70-80 wt% of the total weight of active ingredient in the pharmaceutical composition is present in the immediate release granules and the remaining active ingredient is present in the sustained release pellets. The present inventors have found that this allocation of active ingredient to immediate release granules and sustained release pellets provides a good balance between quickly reaching an effective concentration of the active ingredient in the bloodstream and maintaining the concentration of the active ingredient at or above the efficient concentration between administrations of the pharmaceutical composition.

The step i. of producing the immediate release granules may comprise the substep of mixing active ingredient with filler, followed by the substep of compacting the mixture to form granules. The compaction may preferably be done by slugging and milling, but more preferably by roller compaction. The obtained granules may further be dried. The step ii.. of producing the pellet cores may comprise the substep of mixing the active ingredient with filler, followed by the substep of compacting the mixture to form pellets. The pellets may in particular be produced by extrusion and spheronization. The obtained pellets may preferably be dried. Preferably, the pellets are produced and/or dried so as to have a moisture content of below 5%, preferably, below 3.5%

The step iii... of coating the pellet cores with the subcoat is performed on the produced pellet cores. The step of coating comprises suspending or dissolving the subcoat in aqueous media to form an aqueous solution or dispersion of the subcoat, followed by the substep of contacting the pellet cores with the aqueous solution or dispersion of the subcoat. The contacting may be performed by mixing the pellet cores with the aqueous solution or dispersion of the subcoat. The contacting may be followed by a drying step to dry the pellet cores. The result of coating the pellet cores with the subcoat is pellet cores provided with the subcoat. The step iv. of further coating the pellet cores provided with the subcoat with a sustained release coating comprises the substeps of suspending or dissolving the sustained release coating in aqueous media to form an aqueous solution or dispersion, and contacting the pellet cores, coated with the subcoat, with the aqueous solution or dispersion of the sustained release coating to produce sustained release pellets. As above, the contacting may be performed by mixing the pellet cores coated with the subcoat, with the aqueous solution or dispersion of the sustained release coating. The aqueous media preferably comprises at least 90%, preferably at least 95%, more preferably 99-100% water. Typically, the aqueous solution or dispersion is an aqueous dispersion.

Following step iv, the sustained release pellets were preferably dried to a moisture content below 5%, preferably below 3.5%. This may be done in a fluidized bed at 55-65°C, preferably 50-60°C, for 60-150 min, preferably 80-150 min, more preferably 90-150 min. It is however more preferred to dry the sustained release pellets in an oven at 35-45°C, preferably 40 °C for 18-30, preferably 22-26, such as 24, hours.

Generally, the sustained release pellets do not need to be provided with an enteric coating on top of the sustained release coating.

It is further preferred that the sustained release pellets, after production, are not rewetted in aqueous media since this may result in migration of the active pharmaceutical ingredient through the sustained release coating.

Preferably the amount of active ingredient in the immediate release granules makes up 72-77 wt%, preferably 75 wt%, of the total weight of active ingredient in the pharmaceutical composition, and
the amount of active ingredient in the sustained release pellets makes up 23-27 wt%, preferably 25 wt%, of the total weight of active ingredient in the pharmaceutical composition.

These ranges provide even shorter times between administration of the pharmaceutical composition and attainment of an effective plasma concentration. Further, these ranges provide an even lower maximum plasma concentration.

Preferably the immediate release granules have a maximum diameter of 710-1000 µm, and the sustained release pellets have a maximum diameter of 1250-1700 µm. Here the maximum diameter corresponds to the maximum dimension, e.g., length, width, height, of the immediate release granules and the sustained release pellets.

This range of maximum diameters facilitates the passage of the granules through the stomach in the manner of a liquid, i.e., without being hindered by the activities of the pyloric sphincter.

The immediate release granules and the sustained release pellets may be produced to have these maximum diameters using sieves. A first sieve having openings corresponding to the upper limit of the ranges may be used so as to screen off immediate release granules and the sustained release pellets having maximum diameters above this limit, respectively. A second screen having openings corresponding to the lower limit of the ranges may be used to remove immediate release granules and the sustained release pellets having maximum diameters below this limit, respectively.

Preferably the content of the active ingredient in the immediate release granules is 75-95 wt%, preferably 77-83 wt%, based on the weight of the immediate release granules, and the content of the active ingredient in the pellet cores is 35-45 wt%, preferably 37-43 wt%, based on the weight of the pellet cores.

This is advantageous in that a high content of active ingredient compared to other components, e.g., filler and other excipients, decreases the amount of pharmaceutical composition that has to be administered to obtain the effective concentration.

These ratios and ranges have further been found to provide a good balance between maintaining a high content of active ingredient in the pharmaceutical composition while maintaining a good compactability and formability during production of the immediate release granules and the sustained release pellets.

Preferably the content of the filler in the immediate release granules is at least 5 wt% based on the weight of the immediate release granules, and
the content of the filler in the pellet cores is at least 50 wt% based on the weight of the pellet cores.

These values of filler are preferred in that they help maintain a good compatibility and formability during production of the immediate release granules and the sustained release pellets.

Preferably the filler in the immediate release granules is dicalcium phosphate, and
the filler in the pellet cores is microcrystalline cellulose.

Dicalcium phosphate has been shown to be an effective filler in the immediate release granules where generally the content of filler is low. In contrast, microcrystalline cellulose has been shown to be an effective filler in the pellet cores where the content of filler is high and where the wettability of the microcrystalline cellulose may assist in controlling the release rate of active ingredient during the course of dissolution of the sustained release pellet.

Preferably the immediate release granules further comprise 0.3-1 wt%, preferably 0.5 wt%, lubricant based on the weight of the immediate release granules, and the pellet cores further comprise 0.5-2 wt%, preferably 1 wt%, lubricant based on the weight of the pellet cores.

Adding these amounts of lubricant further facilitates the production of the immediate release granules and the sustained release pellets.

The lubricant is in both cases preferably magnesium stearate.

Preferably the content of the subcoat in the sustained release pellets is 14-18 wt%, preferably 15 wt%, based on the weight of the pellet cores.

This is advantageous in that this amount of subcoat obtains the purpose of preventing wetting of the pellet cores when applying the sustained release coating, while at the same time being low enough so as to not interfere with the release rate determining function of the sustained release coating. The contents cited correspond to coating the pellet cores until the corresponding weight increases are obtained.

Preferably the subcoat comprises, by weight of the subcoat:
i. 35-40 wt%, preferably 37.5 wt%, hydroxypropyl cellulose,
ii. 35-40 wt%, preferably 37.5 wt%, hydroxypropyl methylcellulose, and
iii. 20-30 wt%, preferably 25 wt%, talc

This combination of components has shown to provide a subcoat that has good hydrophobic properties, in part provided by the added talc (hydrated magnesium silicate with the chemical formula Mg₃Si₄O₁₀(OH)₂), which further protect the pellet core during the application of the sustained release coating. In these embodiments the hydroxypropyl cellulose and the hydroxypropyl methylcellulose are film forming agents.

Preferably the sustained release coating comprises, by weight of the sustained release coating:
i. 97-100 wt% film forming agent, and
ii. 0-3 wt% of a pore former comprising or consisting of hydroxypropyl methylcellulose.

These ranges provide a sustained release coating providing a good release profile. Further, a content of pore former of 0-3 wt% assists in adjusting the release rate from the sustained release pellets.

If there are more than one film forming agent, the range of 97-100 wt% film forming agents refers to the total content of film forming agents.

Preferably the film forming agent is ethylcellulose. Preferably the pore former comprises hydroxypropyl methylcellulose.

Preferably the content of the sustained release coating in the sustained release pellets is 65-80 wt%, preferably 68-75 wt%, more preferably 70 wt%, based on the weight of the pellet cores provided with the subcoat.

These ranges have been shown to provide advantageous release rates of the active ingredient. These amounts of sustained release coating correspond to the corresponding weight increases during the application of the sustained release coating on the pellet cores coated with the subcoat.

Preferably:
a) the immediate release granules consist of:
   i. 89-91 wt% valproic acid or valproate,
   ii. 8.5-10.5 wt% dicalcium phosphate anhydrous, and
   iii. 0.4-0.6 wt% magnesium stearate,
b) the pellet cores consist of:
   i. 39-41 wt% valproic acid or valproate,
   ii. 58-60 wt% microcrystalline cellulose, and
   iii. 0.5-1.5 wt% magnesium stearate,
c) the subcoat:
   i. is provided in an amount corresponding to 16-17 % of the weight of the pellet cores, and
   ii. comprises, by weight of the subcoat:
      1) 36.5-38.5 wt% hydroxypropyl cellulose,
      2) 36.5-38.5 wt% hydroxypropyl methylcellulose, and
      3) 24-26 wt% talc, and
d) the sustained release coating:
   i. is provided in an amount corresponding to 69-71 % of the weight of the pellet cores coated with the subcoat, and
   ii. comprises, by weight of the sustained release coating:
      1) 97-99% ethylcellulose, and,
      2) 1-3% of a pore former comprising hydroxypropyl methylcellulose,
      wherein:
      the amount of active ingredient in the immediate release granules makes up 74-76 wt% of the total weight of active ingredient in the pharmaceutical composition, and
      the amount of active ingredient in the sustained release pellets makes up 24-26 wt% of the total weight of active ingredient in the pharmaceutical composition.

More preferably:
a) the immediate release granules consist of:
   i. 90 wt% valproic acid or valproate,
   ii. 9.5 wt% dicalcium phosphate anhydrous, and
   iii. 0.5 wt% magnesium stearate,
b) the pellet cores consist of:
   i. 40 wt% valproic acid or valproate,
   ii. 59 wt% microcrystalline cellulose, and
   iii. 1 wt% magnesium stearate,
c) the subcoat:
   i. is provided in an amount corresponding to 15 % of the weight of the pellet cores, and
   ii. comprises, by weight of the subcoat:
      1) 37.5 wt% hydroxypropyl cellulose,
      2) 37.5 wt% hydroxypropyl methylcellulose, and
      3) 25 wt% talc, and
d) the sustained release coating:
   i. is provided in an amount corresponding to 70 % of the weight of the pellet cores coated with the subcoat, and
   ii. comprises, by weight of the sustained release coating:
      1) 98% ethylcellulose, and,
      2) 2% of a pore former comprising hydroxypropyl methylcellulose,
      wherein:
      the amount of active ingredient in the immediate release granules makes up 75 wt% of the total weight of active ingredient in the pharmaceutical composition, and
      the amount of active ingredient in the sustained release pellets makes up 25 wt% of the total weight of active ingredient in the pharmaceutical composition.

This embodiment generally corresponds to the formulation arrived at in example 1.

Preferably the pharmaceutical composition further comprises a capsule for holding the immediate release granules and the sustained release pellets, the capsule defining a unit dose of the pharmaceutical composition.

Each unit dose of the pharmaceutical composition may comprise 250-285 mg of the active ingredient when the capsule is a size0 capsule. The capsule is preferably, as discussed initially, made from a material such as hydroxypropyl methylcellulose that is eroded in the stomach or intestine to release the immediate release granules and the sustained release pellets. The content of active ingredient in each capsule may typically be selected so that each administration, out of two administration per day, comprises the administration of 5-12, such as 7-10, capsules.

The third aspect of the present invention relates to the pharmaceutical composition according to the first aspect of the present invention for use in a method of pretreating cancer, wherein the pharmaceutical composition is administered to a human suffering from cancer.

An alternative third aspect of the present invention relates to a method of pretreating cancer comprising administering the pharmaceutical composition according to the first aspect of the present invention to a human suffering from cancer.

In the context of the present invention, pretreating cancer encompasses treatment performed to make the cancer more susceptible to further treatment. Pretreating cancer thus comprises where one or more compositions are administered to an individual in need of cancer treatment as a pretreatment prior to other treatments to enhance the effect, or mitigate the side-effects, of the treatments.

Typical doses of the active ingredient may be up to 60 mg per kg bodyweight per day, such as 30-60 mg per kg bodyweight per day.

A typical dose of the active ingredient may be 1500 to 2500 mg, more preferably 1800 to 2200 mg, most preferably 2000 mg, per day.

Preferably the cancer is selected from the croup consisting of diffuse large B cell lymphoma (DLBCL), follicular lymphoma, chronic lymphocytic leukaemia, T cell lymphoma, myeloma, and Hodgkin lymphoma.

As shown by WO2012/128709, these cancer types are especially advantageous to pretreat using the active ingredient.

Preferably the pharmaceutical composition is administered prior to the human being treated with chemotherapy and/or immunotherapy.

The chemotherapy and/or immunotherapy should be selected based on the
type of cancer that the human is suffering from.

The chemotherapy may comprise administration of CHOP, a combination of cyclophosphamide, doxorubicin, vincristine, and prednisone, which may be administered in amounts of 750 +/- 10% mg/m2 of cyclophosphamide, 50 +/- 10% mg/m2 of doxorubicin, 1.4 +/- 10% mg/m2 of vincristine and 50 +/- 10% mg/m2 of prednisone, wherein m2 refers to the body surface of the human.

Alternatively the chemotherapy may comprise administration of R-CHOP, i.e. a combination of the antibody Rituximab and cyclophosphamide, doxorubicin, vincristine, and prednisone.

Each or CHOP and R-CHOP may further comprise etoposide, and the corresponding chemotherapy is thus termed CHOEP or R-CHOEP.

The immunotherapy may comprise administering an antibody, monoclonal antibody, or a functional fragment thereof, such as rituximab, ofatumumab, GA101, tositumumab, ibritumumab, ocraluzumab, veltuzumab, epratuzumab, FTBA05, AME-133V or R603. All the above-mentioned antibodies bind to CD20 present on B-cells. The antibodies may be administrated in an amount of 375 +/- 10% mg/m2.

Other examples of treatment that may follow the pretreating comprise surgery, radiation, and gene therapy.

Preferably the pharmaceutical composition is administered twice daily.

Since the active ingredient is preferably taken together with, or in connection with, food, it is advantageous to administer the pharmaceutical composition twice daily as that provides more possibilities for administration together with meals.

Preferably a steroid selected from the group consisting of prednisone, prednisolone, dexamethasone, and betamethasone, is also administered to the human. The steroid may be administered at the same time as the pharmaceutical composition, or may be administered between administrations of the pharmaceutical composition.

Prednisone or prednisolone may be administered in an amount of 20 to 200 mg per day, such as 50-200, 100-150, 30, 40, 50, 60, 70, 80, 90, 100, 110, 120, 130, 140, 150, 160, 170,180, 190 or 200mg per day. The amount may be administered in one or more doses.

Betamethasone may be administered in an amount of 4 to 32 mg per day, such as 10-25, 10-20, 5, 6, 7, 8, 9, 10, 11, 12, 13, 14, 15, 16, 17, 18, 19, 20, 21, 22, 23, 24, 25, 26, 27, 28, 29, 30, 31 or 32 mg per day. The amount may be administered in one or more doses.

Dexamethasone may be administered in an amount of 10 to 80 mg per day, such as 20-70, 10, 20, 30, 40, 50, 60, 70 or 80 mg. The amount may be administered in one or more doses.

A further aspect of the present invention concerns a kit comprising:
- a number of unit doses of the pharmaceutical composition,
- a number of doses of a steroid selected from the group consisting of prednisone, prednisolone, dexamethasone, and betamethasone.

The kit is useful for providing and maintaining an effective concentration of the active ingredient during one session of pretreating cancer. The one session of pretreating cancer may for example comprise a time period of at least 12 hours such as 24 hours or at least 36 hours or at least 48 hours, and less than 96 hours such as less than 84 hours, such as less than 72 hours.

Further, the kit makes it easier to perform pretreatment of cancer and increases the compliance with the pretreatment.

The kit preferably comprises a number of unit doses sufficient for administration of two or three doses per 24-hour period during the session of pretreating cancer. The number of unit doses in the kit may be from 2 up to 8 doses.

The session of pretreating cancer preferably starts 48 hours prior to the treatment of cancer begins. Typically, there are 6 or 9 unit doses.

At least one of the doses may further comprise a steroid selected from the group consisting of prednisone, prednisolone, dexamethasone, and betamethasone. In this case the steroid does not need to be present in each dose.

In some embodiments of the kit the number of unit doses are provided in a suitable container and/or with suitable packaging, and the kit preferably further comprises instructions for how and when to administer the doses.

Thus, the kit may for example comprise one or more blisters for holding the doses. Preferably the kit comprises a blister pack comprising a plurality of collapsible blisters sealed by a frangible sealing sheet, each of the blisters holding one or more doses.

In some embodiments, the kit comprises a number of unit doses sufficient for a plurality, such as 6, sessions of pretreating cancer. Thus, a single kit may be used for all unit doses needed for a typical cycle of treating cancer consisting of six sessions 14-21 days apart, wherein in each session the pharmaceutical composition is administered as pretreatment prior to other chemotherapy or immunotherapy.

### EXAMPLE 1 - Towards developing a sustained release composition of valproate

### 1. 1 Development of immediate release granule

The immediate release granules were developed to contain the following components, see table 1.

**Table 1**

| Material | Batch quantity (g) | Percentage formula wt% |
|---|---|---|
| Sodium valproate CAS 1069-66-05 (active ingredient) | 1800.0 | 90 |
| Dicalcium phosphate anhydrous (A-Tab anhydrous from Innophos (filler) | 190.0 | 9.5 |
| Magnesium stearate (Ligamed MF2V from Peter Greven) (lubricant) | 10.00 | 0.5 |

The immediate release granules are formed by mixing the materials in a planet blender or high-shear blender. The resulting mixture was roller compacted to form solid ribbons which were milled with a 1 mm size screen to form the granules.

### 1.2 Development of pellet cores

The pellets cores were developed to contain the following components, see table 2:

**Table 2**

| Material | Batch quantity (g) | Percentage formula wt% |
|---|---|---|
| Sodium valproate (CAS 1069-66-05 (active ingredient) | 400 | 40 |
| Microcrystalline cellulose (Avicel PH101 from FMC biopolymer) (filler) | 590 | 59 |
| Magnesium stearate (Ligamed MF2V from Peter Greven) (lubricant) | 10 | 1 |

The pellet cores were manufactured by mixing the materials in a planet blender or high-shear blender. Water was added to the mixture and the resulting moist paste was extruded to form portions of material that was then spheronized and dried to form the pellet cores.

### 1.3 Development of subcoat

During the initial development of the sustained release pellets, the pellet cores were coated with various sustained release aqueous based coating polymers such as Surelease, Eudragit NM30D and AcryCoat. These polymers were designed to provide a release in basic pH conditions of between 8 to 12 hours. However, the combination of the highly soluble API in the core and the swelling nature of microcrystalline cellulose in aqueous media, presented a challenge due to the sustained release coating being too porous. This porosity allowed water to penetrate through the coat into the core causing the core to swell, which was observed visually as swelling and rupturing of the coated core, allowing the API to be released completely within one hour.

Various attempts were made to address this problem. It was surprisingly found that introducing a subcoat provided a successful response to limiting the dissolution of the pellet core in acidic media. Adding a subcoat at 10% weight gain (i.e. 10 wt% based on weight of pellet cores) not only allowed the sustained release coating to function as intended, but it also smoothed out irregularities on the pellet surface.

The initial subcoat was applied as an aqueous formulation containing the following components, see table 3A:

**Table 3A**

| Material | Batch quantity per sublot (g) | % solids = Opadry/Total x100 |
|---|---|---|
| Opadry clear (polyvinyl acetate, from Colorcon) | 150.0 | 8.0 |
| Water | 1725.0 | |
| Total | 1875 | |

The subcoat was applied until a weight increase of 10 wt% relative to the weight of the pellet cores was obtained. The subcoat was further optimized and made more hydrophobic by using hydroxypropyl cellulose and hydroxypropyl methylcellulose and by adding the mineral talc (magnesium silicate). The optimized subcoat contained the following components, see table 3B.

**Table 3B**

| Material | Batch quantity per sublot (g) | Percentage formula wt% |
|---|---|---|
| hydroxypropyl cellulose (Klucel LF from BASF) | 86.625 | 37.5 |
| hydroxypropyl methylcellulose (Methocel E5) | 86.625 | 37.5 |
| Talc (hydrophobic component of subcoat) | 57.750 | 25 |
| Water | 2656.500 | |

The optimized subcoat was provided in an amount corresponding to 14-18%, preferably 15 %, of the weight of the pellet cores. These amounts of subcoat are especially advantageous in preventing the active ingredient from migrating from the pellet core and also provide a smooth surface for the sustained release coating.

### 1.4 Development of sustained release coating

The sustained release coating was applied as an aqueous formulation containing the following components, see table 4:

**Table 4**

| Material | Batch quantity per sublot (g) |
|---|---|
| Surelease Ethylcellulose Dispersion type B (from Colorcon) | 1724.0 (431.0 g of dry solids remain from the dispersion after evaporation during processing) |
| Methocel E6 (pore former) | 8.80 (corresponding to 2 wt% pore former based on weight of dry content of sustained release coating |
| Water | 1525.0 |
| Total | 1875 |

The sustained release coating was applied until a weight increase of at least 25 wt% relative to the weight of the subcoated pellet cores was obtained. Increasing the sustained release coating weight gain from 25% to 35, 60 and 100% showed further decrease in the release rate.

Additionally, reducing the concentration of pore former from 20% to 10, 5 and 0% slowed down the release rate significantly leading to minimal release in acidic media and multihour dissolution in phosphate buffer pH 6.8 media.

Further, it was noted that the drying of the sustained release pellets also impacted the release rate.

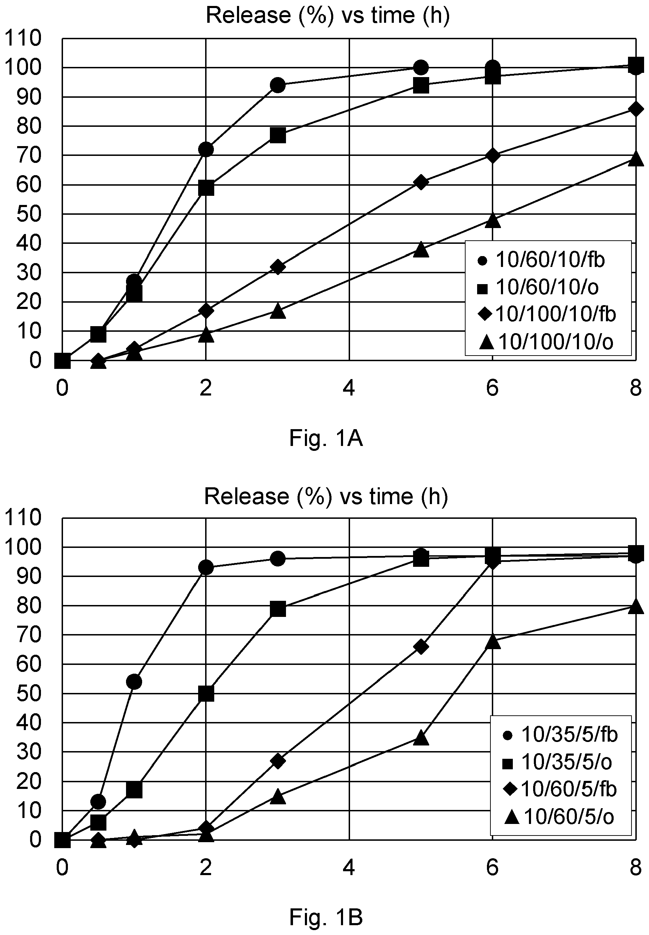

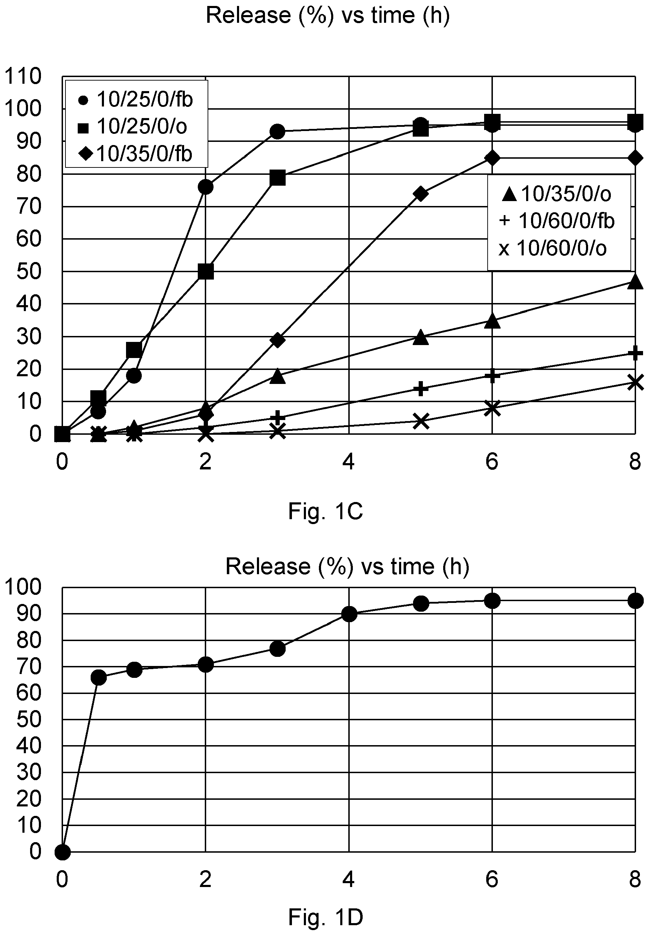

Various pellets were made. Fig. 1A, Fig 1B, and Fig. 1C, show dissolution results for the sustained release pellets with varying amounts of sustained release coating, pore former, and drying/curing method. 2-stage dissolution was used where appropriate to mimic in vivo conditions using 0.1 N HCl for the acidic phase and pH 6.8 phosphate buffer for the alkaline phase as described earlier. The symbols and legends are explained in the following tables:

**Table 5A shows the parameters corresponding to the symbols and legend in Fig. 1A:**

| Symbol | | Subcoat (%wt) | Sustained release coating (%wt) | Pore former (% of sustained release coat) | Drying method |
|---|---|---|---|---|---|
| ● | | 10 | 60 | 10 | fb=Fluid bed (1 h) |
| ■ | | 10 | 60 | 10 | o=Oven (24 h) |
| ◆ | | 10 | 100 | 10 | fb=Fluid bed (1 h) |
| ▲ | | 10 | 100 | 10 | o=Oven (24 h) |

**Table 5B shows the parameters corresponding to the symbols and legend in Fig. 1B:**

| Symbol | Subcoat (%wt) | Sustained release coating (%wt) | Pore former (% of sustained release coat) | Drying method |
|---|---|---|---|---|
| ● | 10 | 35 | 5 | fb=Fluid bed (1 h) |
| ■ | 10 | 35 | 5 | o=Oven (24 h) |
| ◆ | 10 | 60 | 5 | fb=Fluid bed (1 h) |
| ▲ | 10 | 60 | 5 | o=Oven (24 h) |

**Table 5C shows the parameters corresponding to the symbols and legend in Fig. 1C:**

| Symbol | Subcoat (%wt) | Sustained release coating (%wt) | Pore former (% of sustained release coat) | Drying method |
|---|---|---|---|---|
| ● | 10 | 25 | 0 | fb=Fluid bed (1 h) |
| ■ | 10 | 25 | 0 | o=Oven (24 h) |
| ◆ | 10 | 35 | 0 | fb=Fluid bed (1 h) |
| ▲ | 10 | 35 | 0 | o=Oven (24 h) |
| ÷ | 10 | 60 | 0 | fb=Fluid bed (1 h) |
| × | 10 | 60 | 0 | o=Oven (24 h) |

The two stage dissolution data for pellets showed that application of a 10% w/w Opadry sub-coat, including no pore former and up to 60% w/w sustained release Surelease coat, provided a sustained release profile in excess of 12 hours, whereas a thinner coat (35% w/w) release is complete within 6 hours. However, drying/curing the coated pellets at 40°C for 24 hours results in a smoother coat and the release profile was altered-significantly in some cases (35% weight gain, 68% release at 12 hours, 60% weight gain, 31% release at 12 hours). This demonstrated that drying/curing was having an effect on the release profile by allowing the coat to coalesce over time forming less and smaller pores through the coat. Pellets that were coated with 10% and 5% pore former had faster release rates than no pore former. For the pellets coated with Surelease including 10% pore former, 100% was released within 6 hours after applying a 60% weight gain. With a 100% weight gain the release was extended out to more than 12 hours, however, this would result in the coated pellets being too big considering capsule size limitations. For pellets coated with Surelease including 5% pore former, there was 100% release with 35% weight gain within two hours. For the pellets coated with 60% weight gain that were oven dried/cured, full release was not achieved after 12 hours. This data shows that 10% pore former can provide a dissolution profile that is suitable, but the coated pellet will be too large (>1.4 mm) and that with 5% pore former with 60% weight gain, if the right drying/curing time is applied the ideal profile could be met.

### 1.5 Further optimization

The finished sustained release pellets were fluid bed dried/cured at 55° for 1 hour and oven dried/cured at 40°C for 24 hours. There was a significant difference between oven dried and fluid bed dried pellets in terms of release profile. The oven dried pellets performed significantly better by having a slower release rate over 8 or 12 hours.

The highest coating weight gain of each pellet batch (60 or 100%) was further coated with the enteric polymer AcrylEZE (Methacrylic acid copolymer type C). The addition of an enteric coating however did not further improve the release profile.

### 1.6 Development of unit dose capsule

Immediate release granules and sustained release pellets were filled into capsules to a total content of active ingredient of 250 or 285 mg, 75 wt% of the active ingredient being provided by the immediate release granules and 25 wt% of the active ingredient being provided by the sustained release pellets.

### 1.7 Final pharmaceutical composition and dissolution tests

A batch production of a final pharmaceutical composition was made. The pharmaceutical composition was as follows:
Pellet cores as per table 2 were subcoated to 15 wt% weight increase using the optimized subcoat of table 3B. The subcoated pellets were further coated to an 70% weight increase using the sustained release coating of table 4 to produce sustained release pellets of (850-1400 µm) which proceeded to capsule filling.

Immediate release granules according to table 1 and 710-1000 µm were manufactured and proceeded to capsule filling.

Hpmc capsules (size 0EL) were filled with 208.3 mg immediate release granules and 301.5 mg sustained release pellets providing 187.5 mg (75 wt%) immediate release active ingredient and 62.5 mg (25 wt%) sustained release active ingredient. The total amount of active ingredient in the capsules were 250.0 mg.

Fig. 1D shows the 2-stage dissolution profile of the final capsules. As seen in the figure, the capsules of the pharmaceutical composition provide an initially rapid release with about 70% released at 1 hour, followed by a slower release with about 90% released at 4 hours and 95% released at 6 hours.

Samples of the final capsules were further stored under various conditions, e.g., various temperatures (5, 25, and 40°C), humidity (60, 75%RH) for 1 or 3 months. Storing the pharmaceutical formulation under these various conditions did not significantly affect the dissolution profile.

### EXAMPLE 2 - A phase 1, open-label, randomised trial to investigate the pharmacokinetics of the pharmaceutical composition VAL001 under fasting and fed conditions in healthy subjects

In the trial, 25 test subjects were dosed with the final pharmaceutical composition as per 1.7 above, (N=12), termed VAL001 or with a reference composition (N=12), known as Absenor from Orion Pharma.

### 2.1 Single dosing (N=12)

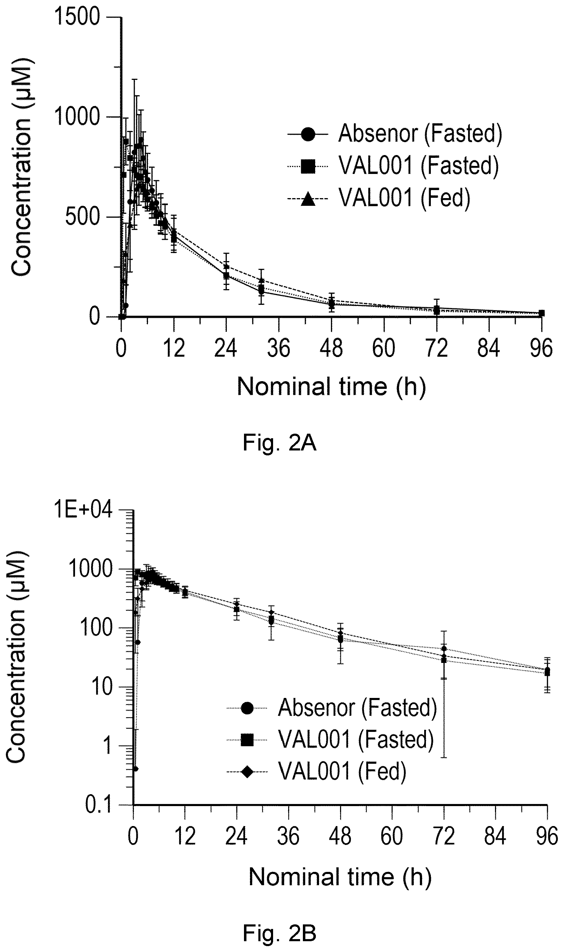

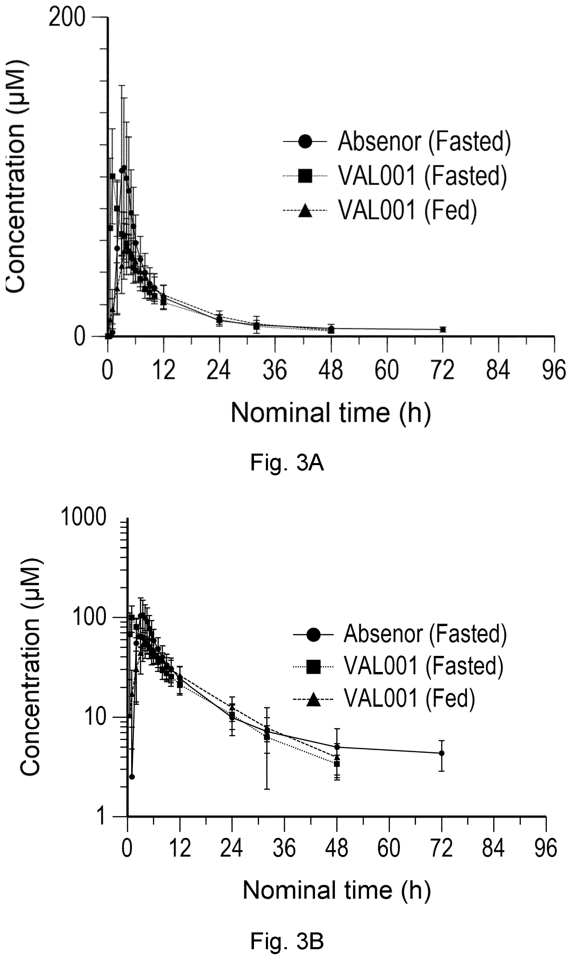

In the first part of the trial, a single dose (corresponding to 30 mg valproate per kg bodyweight) of the final pharmaceutical composition as per 1.7 above (VAL001) in fasted and fed state, and as a single dose of the reference composition (Absenor), was administered to healthy test subjects. Blood samples where then drawn at fixed time points to determine the plasma concentration of valproate. The results are listed in the below table 6 and shown in Fig. 2A-2B and 3A-3B.

**Table 6. Single dosing under fasted conditions**

| Valproate | Parameter | Treatment | n | GLSM (Geometric Least Square Means) | ratio | 90% CI |
|---|---|---|---|---|---|---|
| Total | AUC_{(O-inf)} (h*µM) | VAL001 vs Absenor (fasted | | | 1.08 | 1.01-1.16 |
| | | VAL001 (fasted) | 12 | 15466.5 | | |
| | | Absenor (fasted) | 11 | 14308.0 | | |
| | AUC₍₀₋ₜ₎ (h*µM) | VAL001 vs Absenor (fasted) | | | 1.08 | 1.01-1.14 |
| | | VAL001 (fasted) | 12 | 15017.2 | | |
| | | Absenor (fasted) | 13 | 13945.6 | | |
| | Cₘₐₓ (µM) | VAL001 vs Absenor (fasted) | | | 0.88 | 0.84-0.92 |
| | | VAL001 (fasted) | 12 | 891.51 | | |
| | | Absenor (fasted) | 13 | 1010.72 | | |
| Free | AUC_{(0-inf)} (h*µM) | VAL001 vs Absenor (fasted) | | | 1.03 | 0.95-1-11 |
| | | VAL001 (fasted) | 12 | 935.32 | | |
| | | Absenor (fasted) | 10 | 911.15 | | |
| | AUC₍₀₋ₜ₎ | VAL001 vs Absenor | | | 0.95 | 0.86-1.04 |
| | (h*µM) | (fasted) | | | | |
| | | VAL001 (fasted) | 12 | 859.67 | | |
| | | Absenor (fasted) | 13 | 909.44 | | |
| | Cₘₐₓ(µM) | VAL001 vs Absenor (fasted) | | | 0.78 | 0.70-0.86 |
| | | VAL001 (fasted) | 12 | 101.57 | | |
| | | Absenor (fasted) | 13 | 130.82 | | |

As seen in the table, the VAL001 composition provided a similar exposure but with a lower maximum concentration (Cₘₐₓ). This means a lower peak in concentration and thus a lower risk of side effects, considering the high concentrations that are reached with the doses needed to obtain effect. Thus, the lower peak concentrations are especially advantageous considering that generally, a high dose of valproic acid is necessary to obtain efficient pretreatment of cancer.

This behaviour was also shown in Figs. 2A and 3A with corresponding semi logarithmic graphs 2B and 3B. Specifically, Fig. 2A shows the mean (± standard deviation) plasma concentration of total valproate (µM) vs nominal time (h). As also seen in Fig. 2A and 2B, the VAL001 composition reaches Cₘₐₓ faster than the reference composition, especially in the fasted state.

Correspondingly, Fig. 3A shows the mean (± standard deviation) plasma concentration of free (unbound) valproate (µM) vs nominal time (h).

Together, table 6 and Figs 2A-2B, 3A-3B, show that the VAL001 composition provides a plasma profile that is more efficient for administering valproate for pretreatment of cancer. In other words, the VAL001 composition is especially suitable for reaching a high concentration of valproate relatively quickly but without too high Cₘₐₓ values, and maintaining a high concentration over a dosing period so as to obtain a good synergistic effect of the valproate pretreatment and the following treatment of the cancer.

### 2.2 Double vs triple dosing (N=13)

In the second part of the trial the compositions/formulations were administered either as two doses of 30 mg valproate per body weight (VAL001) or as three doses of 20 mg valproate per kg body weight (Absenor) to the fed healthy test subjects.

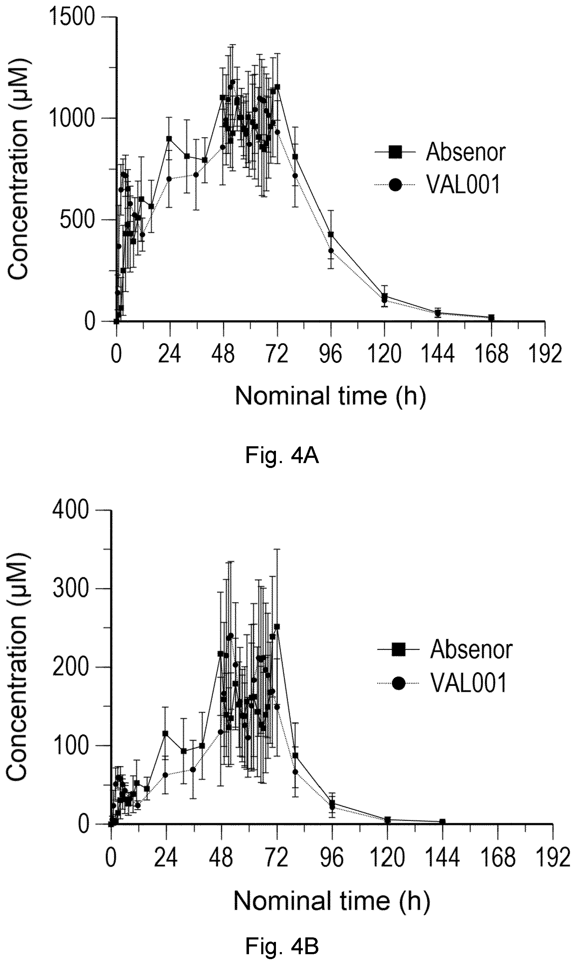

Blood samples where then drawn at fixed time points to determine the plasma concentration of valproate. The results are listed in the below table 7 and shown in Fig. 4A-4B.

**Table 7. Repeated dosing underfed conditions**

| Parameter (unit) | Summary statistic | VAL001 total | Absenor total | VAL001 free | Absenor free |
|---|---|---|---|---|---|
| Cₘₐₓ₋ₗₐₛₜ (uM) | n | 12 | 12 | 12 | 12 |
| | Mean | 1149.417 | 1197.750 | 230.183 | 268.083 |
| | SD | 184.0220 | 119.8970 | 92.5930 | 64.6029 |
| t_{1/2-last} (h) | n | 12 | 12 | 12 | 11 |
| | Mean | 15.233 | 15.570 | 11.487 | 10.821 |
| | SD | 2.2082 | 2.5259 | 2.2610 | 2.8177 |
| AUC₍₀₋ₜ₎₋ₗₐₛₜ (h*uM) | n | 12 | 12 | 12 | 12 |
| | Geometric mean | 33617.60 | 32851.81 | 3642.995 | 3554.262 |
| | CV% | 20.5 | 21.2 | 45.3 | 38.1 |
| AUC_{(0-inf)-last} (h*uM) | n | 12 | 12 | 12 | 11 |
| | Geometric mean | 34038.15 | 33320.39 | 3712.786 | 3726.804 |
| | CV% | 20.4 | 21.4 | 44.2 | 38.0 |
| AUC₄₈₋₁₆₈ (h*uM) | n | 12 | 12 | 12 | 12 |
| | Geometric mean | 45922.78 | 48301.38 | 5649.607 | 5879.575 |
| | CV% | 19.4 | 18.1 | 44.9 | 37.5 |
| R_{acc} AUC-tau | n | 12 | 8 | 12 | 8 |
| | Mean | 1.926 | 2.918 | 4.754 | 7.588 |
| | SD | 0.1833 | 0.8222 | 1.2085 | 3.3178 |
| R_{acc} Cmax-tau | n | 12 | 12 | 12 | 12 |
| | Mean | 1.515 | 2.856 | 3.426 | 11.604 |
| | SD | 0.1660 | 1.8096 | 0.9088 | 12.0272 |
| Cₐᵥ (µM) | n | 12 | 12 | 12 | 12 |
| | Mean | 1022.687 | 958.703 | 183.309 | 169.976 |
| | SD | 175.0602 | 132.3766 | 80.1312 | 46.7550 |

Fig. 4A shows the mean (± standard deviation) plasma concentration of total valproate (µM) vs nominal time (h) for a twice daily dosing for 3 days of 30 mg valproate per kg body weight, whereas Fig. 4B shows the mean (± standard deviation) plasma concentration of free valproate (µM) vs nominal time (h) for a three times daily dosing for 3 days of 20 mg valproate per kg body weight.

Fig. 4A specifically shows that VAL001 provides a more stable concentration profile and exposure to valproate compared to the reference composition Absenor even when given as twice daily doses instead of three daily doses, of the same total daily dose.

A similar result is shown in Fig 4B, which graph further shows that the VAL001 composition gives less accumulation of valproate in the plasma compared to the reference Absenor composition.

### EXAMPLE 3 - Semi-Quantitative Western Blotting assessment of acetylated histone (K9) in cell lysates of isolated peripheral blood mononuclear cells

An analysis was performed by the Bioanalytical laboratory of Turku University, Finland, to determine the extent of histone acetylation in cell lysates of peripheral blood mononuclear cells obtained from test subjects repeatedly dosed with the final pharmaceutical composition as per 1.7 above (termed VAL001) or with a reference composition (Absenor). The histone acetylation was measured by acetylation of lysine 9 of histone H3 (acH3K9) compared to baseline expression.

The samples were obtained from study subjects participating in the clinical trial as described in Example 1. PBMC samples had been collected prior to dosing and 3 times daily during the treatment period in Part II of the clinical trial. The total number of study samples analysed was 96, including 4 time points from 24 study subjects.

For each sample, the experimental signals of acH3K9 were normalized against the loading control GAPDH to correct for loading variation and to confirm that observed changes represented actual differences between samples. The fold changes representing the relative acH3K9 expression of study samples compared to the baseline expression before dosing are comparable between different blots.

### 3. 1 Sample material and control samples

The sample material was frozen cell pellets of human PBMCs delivered at the bottom of cryotubes, which were stored in an upright position. The PBMCs had been isolated from whole blood samples with Cell Preparation Tubes^{™} (CPT, BD Biosciences). The cell pellets had been washed and diluted with phosphate buffered saline (PBS), aliquoted in cryotubes to equal cell concentrations (0.75 - 1.0 × 10⁶ cells per tube), the PBS had been discarded and the cell pellets had been kept stored frozen at nominal - 80 °C before delivery (on dry ice) to the Bioanalytical Laboratory.

PBMC samples had also been collected from each study subject prior to start of dosing of the trial treatment. These samples were used as control samples to determine the baseline acH3K9 protein expression of each study subject. The frozen study samples, 3 aliquots of each (3 × 96 = 288) sample, were received on dry ice from CRST Oy on the 15th of September, 2022. The study samples were subsequently stored at the Bioanalytical Laboratory's freezer at nominal -80 °C.

### 3.2 Summary of trial treatment

The trial treatment of the study subjects, from which the samples were obtained, comprised repeated dosing of either the sustained release composition according to the invention or the reference composition.

### 3.3 Method

The cell pellets were lysed in 200 µl of Laemmli sample buffer containing SDS, a thiol reducing agent β-mercaptoethanol, protease inhibitor (Roche) and phosphatase inhibitor (Roche). The samples were sonicated with the Diagenode Bioruptor system for 10 min in TPX-tubes (Diagenode), heated (95 °C, 5 min), centrifuged at high speed (4°C, 5 min) and the supernatant was separated into a new tube. SDS-polyacrylamide gel electrophoresis (SDS-PAGE) was used for separating the soluble proteins by molecular weight. Before loading, the samples were heated for 5 min at 95 °C.15 µl of each sample, representing approx. 75 000 cells, were loaded per well onto the Any kD^{™} Mini-PROTEAN^{®} TGX^{™} Precast Protein Gels (Bio-Rad).

All analysed sample gels included two lanes of protein standards (Precision Plus Protein Dual Color Standards) for reference of molecular weight. One protein standard lane per gel were overlaid with the ECL channel on the raw data.

After the electrophoresis, proteins were transferred to PVDF membranes with a Semi-Dry Transfer Turbo cell (Bio-Rad) using the "Standard SD" program with a transfer time of 30 min and with constant voltage of 25 V. The transfer quality of each PVDF membrane was ensured with Ponceau S. staining and visual inspection. After destaining, the membranes were blocked with 5 % non-fat milk (Valio) solution in TBST buffer on a plate shaker for 1 h at room temperature (RT).

The primary antibody incubations were performed on a tube roller overnight at + 4 °C.

Dilutions of 1:1000 (0.042 µg/ml) of rabbit anti-acH3K9 antibody (Cell Signaling Technologies) and 1:10 000 (0.1 µg/ml) of rabbit anti-GAPDH antibody (Abcam) were used. For detection of primary antibodies, an anti-rabbit HRP-conjugated secondary antibody (Cell Signaling Technologies) was used: 1:10 000 (0.006 µg/ml) for detection of acH3K9 and 1:3000 (0.020 µg/ml) for detection of GAPDH. All antibodies used were diluted in 1 % non-fat milk in TBST buffer.

The chemiluminescence signals were developed with SuperSignal West Dura Extended Duration Substrate ECL reagents and captured with a CCD camera-based Sapphire imaging system (Azure Biosystems). The signals were quantified with densitometry using Image J version 1.53c, and the analysed lanes, plots of signal densities and measured peak areas were documented.

Measured signals (peak areas) were imported to an Excel file. The acH3(K9) signal was normalized against the loading control GAPDH signal to mathematically correct for unavoidable sample-to-sample and lane-to-lane variation in protein loading. A lane normalization factor was calculated for each lane by dividing the observed GAPDH signal in the lane with the highest observed GAPDH signal in that blot. To calculate the normalized experimental signals for acH3K9, the observed experimental signals were divided with the lane normalization factor of the lane.

### 3.4 Results

The study samples were analysed in twelve (12) separate 10-well SDS-PAGE gels. The quality of all Western blots was good, and no retests were needed.

The digital ECL images with protein markers for reference, plots of signal densities and measured signals (peak areas) were saved and printed as raw data, and visually checked to be of appropriate quality.

The acH3K9 signal was normalized against the housekeeping protein GAPDH expression. The cell / protein loading and antibody dilutions were optimized to ensure successful signal normalization. However, the normalization is assumed to perform best when the differences in protein loading and GAPDH signals are small. Therefore, the results should be combined with visual inspection of the ECL images. Also, it should be noted that normalized acH3K9 signal values are comparable only within the samples on the same blot and should not be compared to signals on other blots. Nevertheless, the reported fold changes, representing the relative acH3K9 expression of study samples compared to the baseline expression before dosing, may well be compared between different blots. The relative acH3K9 expression after administrating valproate (VAL001 or Absenor), measured as the mean of the fold change, was 1.38 (change +38 %) at time point 23.5 h, 1.49 (change + 49 %) at time point 47.5 h and 1.12 (change + 12 %) at time point 80 h. Standard deviation (SD) of the fold change and coefficient of variation % (CV %) of the fold change were 0.50 and 36 % at time point 23.5 h, 0.64 and 43 % at time point 47.5 h, and 0.59 and 53 % at time point 80 h, respectively.

In the VAL001 treatment group, the mean of the fold change was 1.44 at time point 23.5 h (+44 %), 1.69 (change + 69 %) at time point 47.5 h and 1.26 (change + 26 %) at time point 80 h. Standard deviation (SD) of the fold change and coefficient of variation % (CV %) of the fold change were 0.51 and 36 % at time point 23.5 h, 0.79 and 47 % at time point 47.5 h, and 0.74 and 59 % at time point 80 h, respectively.

In the Absenor treatment group, the mean of the fold change was 1.32 (change + 32 %), at time point 23.5 h, 1.29 (change + 29 %) at time point 47.5 h and 0.98 (change - 2 %) at time point 80 h. Standard deviation (SD) of the fold change and coefficient of variation % (CV %) of the fold change were 0.48 and 36 % at time point 23.5 h, 0.34 and 26 % at time point 47.5 h, and 0.33 and 34 % at time point 80 h, respectively. The Mean of the fold change, % Change, Standard deviation (SD) of the fold change and Coefficient of Variation (CV) % were calculated in Excel and reported for each time point separately. The standard deviation of the fold change and the Coefficient of Variation (CV) % of the fold change represent the variation caused by both biological differences between the study subjects, and the variation of the assay, and they should be carefully considered when reporting differences between experimental groups and control samples.

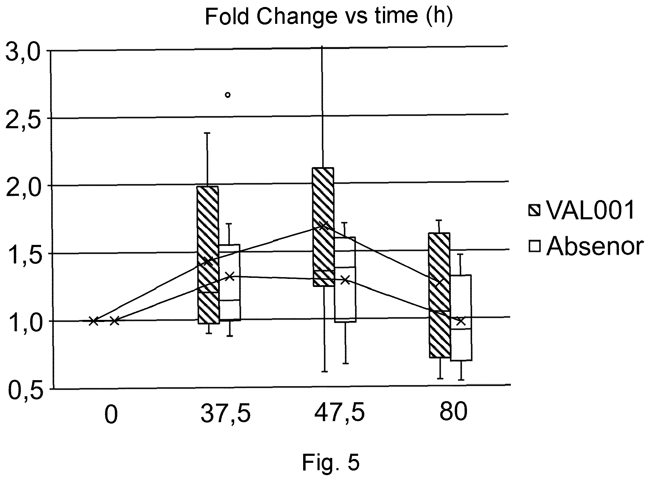

Fig. 5 shows a box plot of the acetylation of lysine 9 In histone H3.

### 2.5 Discussion

As shown by the results, the final pharmaceutical composition as per 1.7 above (VAL001) provided a higher extent of histone acetylation, and thus a more efficient histone deacetylation inhibiting effect than the reference composition (Absenor). The pharmaceutical composition according to the present invention thus provides a more efficient pretreatment of cancer.

## Claims

1. A pharmaceutical composition for oral administration, the pharmaceutical composition comprising:
a) immediate release granules comprising:
i. an active ingredient selected from the group consisting of valproic acid, valproate semisodium, sodium valproate, and magnesium valproate, and
ii. a filler,
b) sustained release pellets, comprising:
i. pellet cores comprising:
(1) an active ingredient selected from the group consisting of valproic acid, valproate semisodium, sodium valproate, and magnesium valproate, and
(2) a filler,
ii. a subcoat provided on the pellet cores, the content of the subcoat being 10-20 wt% based on the weight of the pellet cores, and the subcoat comprising a film forming agent, and
iii. a sustained release coating provided on the subcoat, the content of the sustained release coating being 25-100 wt% based on the weight of the pellet cores coated with the subcoat, and the sustained release coating comprising a film forming agent,
wherein the amount of active ingredient in the immediate release granules makes up 70-80 wt% of the total weight of active ingredient in the pharmaceutical composition, and the amount of active ingredient in the sustained release pellets makes up 20-30 wt% of the total weight of active ingredient in the pharmaceutical composition.

2. The pharmaceutical composition according to claim 1, wherein:
the amount of active ingredient in the immediate release granules makes up 72-77 wt%, preferably 75 wt%, of the total weight of active ingredient in the pharmaceutical composition, and
the amount of active ingredient in the sustained release pellets makes up 23-27 wt%, preferably 25 wt%, of the total weight of active ingredient in the pharmaceutical composition.

3. The pharmaceutical composition according to any preceding claim, wherein:
the content of the active ingredient in the immediate release granules is 75-95 wt%, preferably 77-83 wt%, based on the weight of the immediate release granules, and the content of the active ingredient in the pellet cores is 35-45 wt%, preferably 37-43 wt%, based on the weight of the pellet cores.

4. The pharmaceutical composition according to any preceding claim, wherein:
the content of the subcoat in the sustained release pellets is 14-18 wt%, preferably 15 wt%, based on the weight of the pellet cores.

5. The pharmaceutical composition according to any preceding claim, wherein:
the subcoat comprises, by weight of the subcoat:
i. 35-40 wt%, preferably 37.5 wt%, hydroxypropyl cellulose,
ii. 35-40 wt%, preferably 37.5 wt%, hydroxypropyl methylcellulose, and
iii. 20-30 wt%, preferably 25 wt%, talc

6. The pharmaceutical composition according to any preceding claim, wherein
the sustained release coating comprises, by weight of the sustained release coating:
1) 97-100 wt% film forming agent, and
2) 0-3 wt% of a pore former.

7. The pharmaceutical composition according to any preceding claim, wherein
the content of the sustained release coating in the sustained release pellets is 65-80 wt%, preferably 68-75 wt%, more preferably 70 wt%, based on the weight of the pellet cores provided with the subcoat.

8. The pharmaceutical composition according to any preceding claim, wherein
a) the immediate release granules consist of:
i. 90 wt% valproic acid or valproate,
ii. 9.5 wt% dicalcium phosphate anhydrous, and
iii. 0.5 wt% magnesium stearate,
b) the pellet cores consist of:
i. 40 wt% valproic acid or valproate,
ii. 59 wt% microcrystalline cellulose, and
iii. 1 wt% magnesium stearate,
c) the subcoat:
i. is provided in an amount corresponding to 15 % of the weight of the pellet cores, and
ii. comprises, by weight of the subcoat:
1) 37.5 wt% hydroxypropyl cellulose,
2) 37.5 wt% hydroxypropyl methylcellulose, and
3) 25 wt% talc, and
d) the sustained release coating:
i. is provided in an amount corresponding to 70 % of the weight of the pellet cores coated with the subcoat, and
ii. comprises, by weight of the sustained release coating:
1) 98% ethylcellulose, and,
2) 2% of a pore former comprising hydroxypropyl methylcellulose.
wherein:
the amount of active ingredient in the immediate release granules makes up 75 wt% of the total weight of active ingredient in the pharmaceutical composition, and
the amount of active ingredient in the sustained release pellets makes up 25 wt% of the total weight of active ingredient in the pharmaceutical composition.

9. The pharmaceutical composition according to any preceding claim, further comprising a capsule for holding the immediate release granules and the sustained release pellets, the capsule defining a unit dose of the pharmaceutical composition.

10. A method of producing the pharmaceutical composition according to any preceding claim, comprising the steps of:
i. producing the immediate release granules,
ii. producing the pellet cores,
iii. coating the pellet cores with the subcoat by performing the substeps of:
a. suspending or dissolving the subcoat in aqueous media to form an aqueous solution or dispersion of the subcoat,
b. contacting the pellet cores with the aqueous solution or dispersion of the subcoat to produce pellet cores provided with the subcoat,
iv. further coating the pellet cores provided with the subcoat with a sustained release coating by performing the substeps of
a. suspending or dissolving the sustained release coating in aqueous media to form an aqueous solution or dispersion of the sustained release coating, and
b. contacting the pellet cores, coated with the subcoat, with the aqueous solution or dispersion of the sustained release coating to produce sustained release pellets.

11. The pharmaceutical composition according to any of the claims 1-10 for use in a method of pretreating cancer, wherein the pharmaceutical composition is administered to a human suffering from cancer.

12. The pharmaceutical composition for use according to claim 11, wherein the cancer is selected from the croup consisting of diffuse large B cell lymphoma (DLBCL), follicular lymphoma, chronic lymphocytic leukaemia, T cell lymphoma, myeloma, and Hodgkin lymphoma.

13. The pharmaceutical composition for use according to any of claims 11-12, wherein the pharmaceutical composition is administered prior to the human being treated with chemotherapy and/or immunotherapy.

14. The pharmaceutical composition for use according to any of claims 11-13, wherein the pharmaceutical composition is administered twice daily.

15. The pharmaceutical composition for use according to any of claims 11-14, wherein a steroid selected from the group consisting of prednisone, prednisolone, dexamethasone, and betamethasone, is also administered to the human.
